# FASCICULE DE BREVET EUROPEEN

(11) **EP 0 278 876 B1**
(45) Date de publication et mention de la délivrance du brevet: **23.11.1994**
(21) Numéro de dépôt: 88420026.2
(22) Date de dépôt: 28.01.1988
(51) Int. Cl.: A61K 39/395, A61K 9/20, A61K 9/18

(54) **Formes galéniques de gammaglobulines pour administration par voie sublinguale**
Galenische Formen von Gammaglobulinen für die sublinguale Verabreichung
Galenical forms of gammaglobulins for sublingual administration

(30) Priorité: 28.01.1987 FR 8701380
(43) Date de publication de la demande: 17.08.1988
(73) Titulaire: MEDIBREVEX SA, 74940 Annecy Le Vieux (FR)
(72) Inventeur: Bruttmann, Georges, F-38000 Grenoble (FR); Pedrali, Patrick, F-74000 Annecy le Vieux (FR); Robert, Serge, Braine le Chateau (BE)
(74) Mandataire: Maureau, Philippe

(56) Documents cités:
- EP-A- 0 094 575
- FR-A- 2 460 137
- G. NETIEN et al., "Galenica 16 - Médicaments homéopathiques", édition 2, 1986, Technique et Documentation, Paris (FR); pp. 77-99#

## Description

La présente invention concerne des formes galéniques de gammaglobulines pour administration par voie sublinguale.

On sait que, à côté des techniques d'immunothérapie active et spécifique utilisant des allergènes pour les traitements de désensibilisation, il existe des techniques d'immunothérapie passive utilisant aussi bien des gammaglobulines globales, non spécifiques, que des gammaglobulines spécifiques.

Des travaux de Bernstein et Al (the Jal of Immunology - vol. 120, n°2, 658-654, 1978, Int. Archs. Allergy appl. Immun. 58 : 30-37, 1979) ont démontré que l'injection d'anticorps spécifiques anti-pollens de ragweed protégeait le malade allergique à ce pollen et modifiait son comportement immunitaire.

Ces gammaglobulines, sous forme de solutés, sont appliquées le plus souvent par injection intramusculaire ou sous cutanée, et l'on connaît les problèmes que pose ce mode d'application douloureux et très souvent suivi de réactions locales, voire générales ; ce mode d'application est mal accepté par le patient qui en vient souvent à refuser la poursuite du traitement.

D'autre part, toutes les gammaglobulines potentiellement utilisables ne sont pas disponibles sous forme galénique convenant à l'injection.

Le document EP-A-0 094 575 décrit une forme galénique liquide d'administration par la voie sublinguale d'un mélange constitué par de l'histamine et une immunoglobuline. L'histamine provoque la dilatation de différents tissus ou capillaires de l'organisme, et partant favorise le passage de l'immunoglobuline seule au travers de la muqueuse sublinguale. Au vu de ce document, rien ne permet d'indiquer ou de prévoir qu'une gammaglobuline puisse être administrée seule par la voie sublinguale. Et ce médicament requiert l'adjonction d'histamine dont les effets secondaires ne sont pas négligeables.

Il serait donc important de pouvoir disposer de nouvelles formes galéniques de gammaglobulines, spécifiques ou non spécifiques, qui ne présentent pas les inconvénients exposés ci-avant.

Les inventeurs ont, au cours de leurs études, réussi à mettre au point de nouvelles formes galéniques permettant l'application de ces gammaglobulines par voie sublinguale. On dispose ainsi d'un mode d'application totalement indolore, donc mieux toléré par le patient, ce qui permet, comme on le verra par la suite, d'élargir de façon importante les possibilités d'utilisation thérapeutique de ces produits.

Une composition immunothérapique selon l'invention, se caractérise par le fait que les gammaglobulines sont contenues, en quantités strictement contrôlées et reproductibles, dans un support solide pharmaceutiquement acceptable, adapté à une administration sublinguale.

Conformément au document suivant : "Galenica 16- Médicaments homéopathiques, édition 2, 1986, pages 77-99, Technique et Documentation, Paris, France (G Netien)", et pour des médicaments homéopathiques, on sait déjà obtenir des formes galéniques solides pour le support de différentes substances, aux fins d'une administration sublinguale. Mais en homéopathie, les différents principes actifs utilisés sont présents sur le support solide, en quantités infinitésimales, voire nulles, lesquelles en tous cas ne peuvent plus être dosées, et par conséquent contrôlées.

Il est donc possible d'utiliser des gammaglobulines non injectables en début de désensibilisation active ; on peut aussi, grâce à ces nouvelles formes galéniques, utiliser par voie sub-linguale des gammaglobulines dans les échecs de désensibilisation par voie injectable ; on a en plus la possibilité d'apporter une immunité renforcée en IgG à partir du 7ème mois chez les femmes enceintes, ce qui présente un intérêt tout particulier puisque l'on sait que cette immunité sera transplacentaire, donc transmise ; on peut ainsi éviter au nouveau-né les inconvénients des trois premiers mois de la vie où il se trouve en hypogammaglobulinémie (IgG) physiologique.

Le procédé de préparation des nouvelles formes galéniques de gammaglobulines pour application par voie sub-linguale va maintenant être décrit en détail.

Le produit de départ est constitué de gammaglobulines sous forme solide et de préférence lyophilisée.

Les inventeurs ont pu démontrer qu'il n'existait pas de différence importante, quant à la teneur en anticorps spécifiques anti-allergiques, dans les différentes préparations de gammaglobulines commercialisées, ce qui permet d'utiliser, en immunothérapie passive, aussi bien les gammaglobulines d'origine plasmatique que les gammaglobulines d'origine placentaire. Il est donc possible de partir indifféremment de l'une ou l'autre source.

Il a par ailleurs semblé spécialement intéressant aux inventeurs d'envisager l'emploi des anticorps, non plus en mélange, comme c'est le cas dans les formes galéniques injectables, mais après isolation, en tant qu'anticorps monospécifiques.

Le placenta humain offrant un mélange d'anticorps anti-allergiques voisin de ce que contient le plasma humain, on utilise de préférence ce placenta comme source d'anticorps, ce qui présente également des avantages importants en matière de prix de revient.

On effectue donc un broyage du placenta qui permet d'obtenir un mélange d'anticorps comprenant surtout des immunoglobulines de la classe des IgG, tels que :
- anticorps anti-pollens de graminées,
- anticorps anti-poussière de maison et acariens,
- anticorps anti-venin de guêpes,
- anticorps anti-venin d'abeilles.

Ce mélange d'anticorps renferme également des anticorps anti-viraux qui pourront être isolés par ailleurs.

On fait passer le mélange d'anticorps anti-allergiques sur des colonnes de verre afin de séparer les anticorps selon le processus suivant :

Passage sur une première colonne de verre contenant un anti-IgG de pollen.

Il reste un mélange dépourvu d'anticorps anti-pollens de graminées.

Le passage de ce mélange sur une deuxième colonne de verre contenant un anticorps anti-poussière-acariens retient les anticorps de ce type.

Les anticorps anti-venins sont ensuite successivement retenus sur deux autres colonnes de verre anti-guêpes et anti-abeilles.

Les anticorps monospécifiques ainsi obtenus sont mis sous forme lyophilisée.

Le produit de départ est donc constitué de l'un (ou de plusieurs) de ces anticorps sous forme lyophilisée que l'on met en solution, dans tout solvant convenable de façon à obtenir une solution-mère de concentration élevée.

Le solvant utilisé peut être l'eau ou le sérum physiologique.

On prépare ensuite, en utilisant le même solvant que celui qui a servi à obtenir la solution-mère des dilutions permettant l'imprégnation de 2, 3,5, ou même 10 mg de gammaglobulines par gélule contenant de 400 à 700 mg de globules imprégnés.

Toutes les précautions seront prises, lors de chacune de ces dilution pour que la quantité de solvant porteur de gammaglobulines reste constante, ceci afin de réaliser toutes les imprégnations de façon identique.

On réalise ensuite, dans une turbine, à l'aide d'un injecteur du type dénommé"spray-doseur", l'imprégnation, à l'aide de chacune des dilutions de globules constitués, de façon connue en soi, d'un support solide pharmaceutiquement acceptable et spécialement d'un mélange saccharose-lactose. Il est bien évident que, sans sortir du cadre de l'invention, ces globules pourraient être remplacés par des poudres ou des comprimés, également adaptés à l'application par voie per-linguale ou sub- linguale. La technique d'imprégnation est celle de la multi-imprégnation ou de l'imprégnation fractionnée, de manière à garantir une parfaite répartition du principe actif homogène.

Entre chaque imprégnation, le séchage est réalisé par passage d'air forcé et desséché dans une chambre où règne une dépression obtenue par la différence de débit existant entre ce passage d'air et un puissant extracteur.

Selon une caractéristique importante de l'invention, cette opération de séchage sera effectuée à une température inférieure ou égale à 30°C.

On a, en effet, déterminé, au cours des nombreux essais ayant conduit à la mise au point dudit procédé, que le procédé n'est plus fiable quand la température dépasse 38-40°C.

La dernière imprégnation est une imprégnation protectrice du type "dragéification".

Selon un autre mode de réalisation du procédé selon l'invention, et afin de garantir que soit totalement maintenue l'intégralité physicochimique du principe actif, les différentes étapes de l'imprégnation sont effectuées sous atmosphère d'azote.

La dernière opération consiste à mettre en gélules les globules ainsi obtenus, ou éventuellement à placer lesdits globules ou les poudres ou comprimés dans des tubes-doses et à terminer le conditionnement de façon classique (blisters pour les gélules, coffrets pour les tubes-doses) en numérotant, bien évidemment, les gélules à doses croissantes.

Les nouvelles formes galéniques selon l'invention présentent, - comme expliqué ci-avant, un intérêt pour l'application de l'ensemble des gammaglobulines, qu'il s'agisse de gammaglobulines globales non spécifiques ou de gammaglobulines spécifiques.

Quatre fractions ont fait plus spécialement l'objet d'une étude clinique.

Il s'agit tout d'abord des immuno-globulines non spécifiques totales avec des dosages respectifs de 2, 3 et 5 mg d'immuno-globulines par dose. Les nouvelles formes galéniques selon l'invention permettent de remplacer purement et simplement l'usage des gammaglobulines injectables actuelles, par une voie indolore, donc mieux tolérée, ce qui élargira les indications, compte-tenu de la facilité d'emploi de la nouvelle forme galénique ; on améliorera également le suivi du traitement.

Il s'agit ensuite :
- des immuno-globulines spécifiques anti-pollens de graminées,
- des gammaglobulines anti-poussière de maison et d'acariens,
- des gammaglobulines anti-venin de guêpes et d'abeille.

Les dosages de ces gammaglobulines spécifiques sont identiques à ceux des gammaglobulines spécifiques globales.

L'isolation des anticorps antiviraux semble par ailleurs pleine de promesses.

Il faut souligner une fois encore que l'intérêt majeur de l'invention repose sur la substitution des formes actuelles par la forme sub-linguale. Cette nouvelle voie permet d'utiliser des gammaglobulines non injectables en début de désensibilisation active, d'utiliser par cette même voie des gammaglobulines dans les échecs des désensibilisations faites par la méthode injectable.

On a de même, comme on l'a déjà mentionné ci-avant, la possibilité d'apporter une immunité renforcée en IgG à partir du 7ème mois chez les femmes enceintes, sachant que cette immunothérapie sera transplacentaire, donc transmise.

On évite ainsi tous les inconvénients de la forme injectable : on sait par ailleurs que la voie sub-linguale est idéale pour l'enfant ainsi que pour l'adulte.

La voie sub-linguale permet une absorption très rapide, ce qui permet éventuellement de prévoir des capsules faiblement dosées ; on peut prévoir des capsules à doses croissantes pour les traiements initiaux et des capsules à doses fixes pour les traitements d'entretien.

Du point de vue production industrielle, l'extraction placentaire se révèle fort intéressante.

Il est enfin souvent préférable de proposer une thérapeutique substitutive monospécifique en fonction de la maladie à traiter et non pas un mélange d'anticorps.

Les nouvelles formes galéniques selon l'invention doivent en outre permettre de découvrir la biodisponibilité des gammaglobulines par voie sub-et per-linguale ; rappelons que la biodisponibilité des gammaglobulines injectées est inconnue à ce jour.

Il est bien évident que la présente invention ne saurait se limiter aux concentrations, modes de fractionnement ou dilutions donnés ci-avant à titre d'exemple non limitatif ; ces concentrations, modes de fractionnement et dilutions peuvent, bien entendu, être adaptés sur demande : de même le support saccharose-lactose peut être remplacé par un autre support pharmaceutiquement acceptable adapté.

## Revendications (Revendications pour l'(les) Etat(s) contractant(s) suivant(s): AT, BE, CH, DE, FR, GB, IT, LI, LU, NL, SE)

1. Composition immunothérapique comprenant des gammaglobulines, caractérisée en ce que lesdites gammaglobulines sont contenues, en quantités strictement contrôlées et reproductibles, dans un support solide pharmaceutiquement acceptable, adapté à une administration sublinguale.

2. Composition selon la revendication 1, caractérisée en ce que les gammaglobulines sont sous forme lyophilisée dans le support solide.

3. Composition selon la revendication 1, caractérisée en ce que le support solide est un mélange saccharose-lactose.

4. Composition selon la revendication 1, caractérisée en ce que les gammaglobulines sont obtenues à partir d'un broyat de placenta humain.

5. Composition selon la revendication 1, caractérisée en ce que les gammaglobulines sont d'origine plasmatique.

6. Composition selon la revendication 1, caractérisée en ce que les gammaglobulines sont de la classe IgG.

7. Composition selon la revendication 1, caractérisée en ce que les gammaglobulines sont un mélange de différents anticorps.

8. Composition selon la revendication 1, caractérisée en ce que les gammaglobulines sont un mélange d'anticorps choisis parmi les anticorps anti-pollens de graminées, les anticorps anti-poussière de maison et acariens, les anticorps anti-venin de guêpes, les anticorps anti-venin d'abeilles.

9. Composition selon la revendication 1, caractérisée en ce que les gammaglobulines contiennent un seul anticorps monospécifique.

## Revendications (Revendications pour l'(les) Etat(s) contractant(s) suivant(s): ES, GR)

1. Procédé d'obtention d'une composition immunothérapique comprenant des gammaglobulines, caractérisé en ce qu'on introduit lesdites gammaglobulines, en quantités strictement contrôlées et reproductibles, dans un support solide pharmaceutiquement acceptable, adapté à une administration sublinguale.

2. Procédé selon la revendication 1, caractérisé en ce que les gammaglobulines sont introduites sous forme lyophilisée dans le support solide.

3. Procédé selon la revendication 1, caractérisé en ce que le support solide est un mélange saccharose-lactose.

4. Procédé selon la revendication 1, caractérisé en ce que les gammaglobulines sont obtenues à partir d'un broyat de placenta humain.

5. Procédé selon la revendication 1, caractérisé en ce que les gammaglobulines sont d'origine plasmatique.

6. Procédé selon la revendication 1, caractérisé en ce que les gammaglobulines sont de la classe IgG.

7. Procédé selon la revendication 1, caractérisé en ce que les gammaglobulines sont un mélange de différents anticorps.

## Claims (Claims for the following Contracting State(s): AT, BE, CH, DE, FR, GB, IT, LI, LU, NL, SE)

1. Immunotherapeutic composition comprising gamma-globulins, characterized in that the said gamma-globulins are contained, in strictly controlled and reproducible quantities, in a pharmaceutically acceptable solid vehicle suited to sublingual administration.

2. Composition according to Claim 1, characterized in that the gamma-globulins are in lyophilized form in the solid vehicle.

3. Composition according to Claim 1, characterized in that the solid vehicle is a sucrose/lactose mixture.

4. Composition according to Claim 1, characterized in that the gamma-globulins are obtained from a ground preparation of human placenta.

5. Composition according to Claim 1, characterized in that the gamma-globulins are of plasma origin.

6. Composition according to Claim 1, characterized in that the gamma-globulins are of the IgG class.

7. Composition according to Claim 1, characterized in that the gamma-globulins are a mixture of different antibodies.

8. Composition according to Claim 1, characterized in that the gamma-globulins are a mixture of antibodies chosen from antibodies to grass pollens, antibodies to house dust and mite dust, antibodies to wasp venom and antibodies to bee venom.

9. Composition to Claim 1, characterized in that the gamma-globulins contain a single monospecific antibody.

## Claims (Claims for the following Contracting State(s): ES, GR)

1. Process for obtaining an immunotherapeutic composition comprising gamma-globulins, characterized in that the said gamma-globulins are introduced, in strictly controlled and reproducible quantities, into a pharmaceutically acceptable solid vehicle suited to sublingual administration.

2. Process according to Claim 1, characterized in that the gamma-globulins are introduced in lyophilized form into the solid vehicle.

3. Process according to Claim 1, characterized in that the solid vehicle is a sucrose/lactose mixture.

4. Process according to Claim 1, characterized in that the gamma-globulins are obtained from a ground preparation of human placenta.

5. Process according to Claim 1, characterized in that the gamma-globulins are of plasma origin.

6. Process according to Claim 1, characterized in that the gamma-globulins are of the IgG class.

7. Process according to Claim 1, characterized in that the gamma-globulins are a mixture of different antibodies.

## Patentansprüche (Patentansprüche für folgende(n) Vertragsstaat(en): AT, BE, CH, DE, FR, GB, IT, LI, LU, NL, SE)

1. Immunotherapeutische Zusammensetzung, enthaltend Gammaglobuline, dadurch gekennzeichnet, daß die Gammaglobuline in genau kontrollierten und reproduzierbaren Mengen in einem pharmazeutisch akzeptablen festen Träger enthalten sind, der für eine sublinguale Verabreichung geeignet ist.

2. Zusammensetzung nach Anspruch 1, dadurch gekennzeichnet, daß die Gammaglobuline in lyophilisierter Form im festen Träger enthalten sind.

3. Zusammensetzung nach Anspruch 1, dadurch gekennzeichnet, daß der feste Träger eine Saccharose-Lactose-Mischung ist.

4. Zusammensetzung nach Anspruch 1, dadurch gekennzeichnet, daß die Gammaglobuline aus einer zermalmten Masse an menschlicher Placenta erhalten worden sind.

5. Zusammensetzung nach Anspruch 1, dadurch gekennzeichnet, daß die Gammaglobuline plasmatischen Ursprungs sind.

6. Zusammensetzung nach Anspruch 1, dadurch gekennzeichnet, daß die Gammaglobuline von der Klasse IgG sind.

7. Zusammensetzung nach Anspruch 1, dadurch gekennzeichnet, daß die Gammaglobuline eine Mischung an verschiedenen Antikörpern sind.

8. Zusammensetzung nach Anspruch 1, dadurch gekennzeichnet, daß die Gammaglobuline eine Mischung an Antikörpern sind, ausgewählt aus den Antikörpern gegen Graspollen, den Antikörpern gegen Hausstaub und Milben, den Antikörpern gegen Wespengift, den Antikörpern gegen Bienengift.

9. Zusammensetzung nach Anspruch 1, dadurch gekennzeichnet, daß die Gammaglobuline einen einzigen monospezifischen Antikörper enthalten.

## Patentansprüche (Patentansprüche für folgende(n) Vertragsstaat(en): ES, GR)

1. Verfahren zum Herstellen einer immunotherapeutischen Zusammensetzung, die Gammaglobuline enthält, dadurch gekennzeichnet, daß die Gammaglobuline in genau kontrollierten und reproduzierbaren Mengen in einen pharmazeutisch akzeptablen festen Träger eingebracht werden, der für eine sublinguale Verabreichung geeignet ist.

2. Verfahren nach Anspruch 1, dadurch gekennzeichnet, daß die Gammaglobuline in lyophilisierter Form in den festen Träger eingebracht werden.

3. Verfahren nach Anspruch 1, dadurch gekennzeichnet, daß der feste Träger eine Saccharose-Lactose-Mischung ist.

4. Verfahren nach Anspruch 1, dadurch gekennzeichnet, daß die Gammaglobuline aus einer zermalmten Masse an menschlicher Placenta erhalten worden sind.

5. Verfahren nach Anspruch 1, dadurch gekennzeichnet, daß die Gammaglobuline plasmatischen Ursprungs sind.

6. Verfahren nach Anspruch 1, dadurch gekennzeichnet, daß die Gammaglobuline von der Klasse IgG sind.

7. Verfahren nach Anspruch 1, dadurch gekennzeichnet, daß die Gammaglobuline eine Mischung an verschiedenen Antikörpern sind.
